# EUROPEAN PATENT APPLICATION

(11) **EP 1 712 224 A1**
(43) Date of publication of application: **18.10.2006**
(21) Application number: 05405290.7
(22) Date of filing: 12.04.2005
(51) Int. Cl.: A61K 9/20

(54) **Divisible rectangular flat tablet**

(71) Applicant: von Mérey, Alexander, 2608 Courtelary (CH)
(72) Inventor: von Mérey, Alexander, 2608 Courtelary (CH)

(57) **Abstract**

The invention provides a flat rectangular tablet bevelled and with round edges with directly opposite's grooves.

This allows an easy breaking into two equal parts.

The breakable connecting structure is formed by depressions in the top and bottom facing surfaces. Each of the depressions is directly opposite the other.

The tablets have bevelled edges and rounded corners.

The dimensions and design of this flat tablet are described in the claim.

## Description

### Technical Field

The present invention relates to the novel dosage form that allows a automatic separation of flat tablets with two oppsites grooves.

### Backround of the invention

This tablet form is unique as in fact it shows two opposite grooves and bevelled edges in a rectangualar shape allowing the use of automatic dispensers.

### Summary of the invention

The present invention is directed to a tablet, flat rectangular shape, with bevelled edges and opposites grooves for a dietetic and pharmaceutical formulation in a oral application.

### Brief description of the drawings:

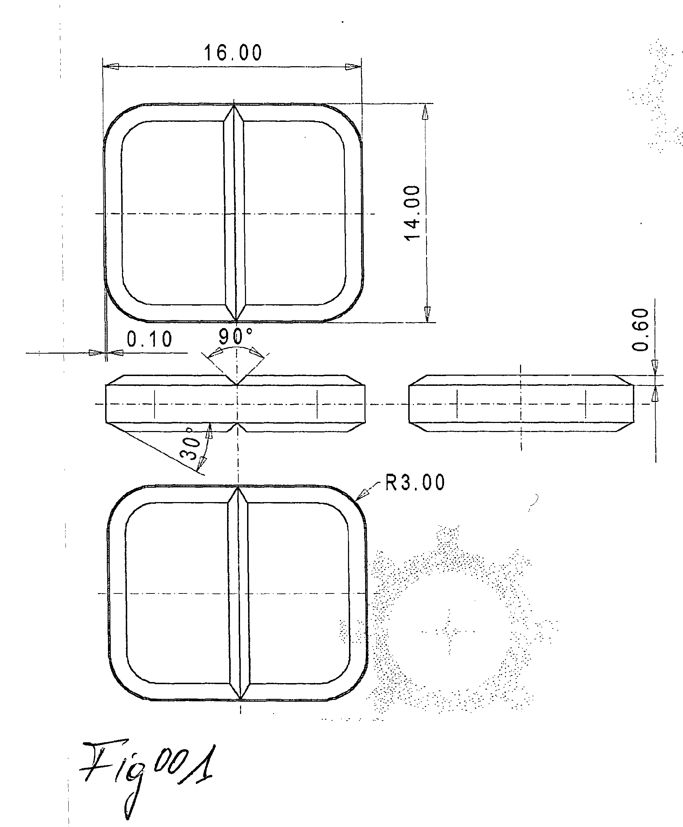

Fig 1 shows a plan view of the flat tablet with opposites groove and bevelled edges and round corners.

### Detailed description of the present invention

The present invention is directed to a novel generally rectangular flat tablet with bevelled edges and 2 opposites grooves.
This shape allows an easy separation of piled tablets in a dispenser.

## Claims

1. A new rectangular flat tabletform with opposites groove and bevelled edges for an easy piling in dispensers for dietetic foods an pharmaceutical products.
The Dimesions are:
**Primo:**
16 mm long, 14 mm large, Radius of the comers: 3 mm, Groove 90°, Side :0.10 mm. Thickness: 4 to 6 mm
**Secundo:**
range of dimensions::
8 to 25 mm long, 7 bis 22 mm large, Radius of the corners 2 to 4 mm, groove 80 to 110 °, Sides 0.07 bis 0.2 mm. Thickness 3 to 10 mm.
